# EUROPÄISCHE PATENTSCHRIFT

(11) **EP 4 076 293 B1**
(45) Veröffentlichungstag und Bekanntmachung des Hinweises auf die Patenterteilung: **28.01.2026**
(21) Anmeldenummer: 20838922.1
(22) Anmeldetag: 15.12.2020
(51) Int. Cl.: A61F 2/76, A61F 2/80, A61F 2/50

(54) **PROTHESENSCHAFT UND VERFAHREN ZUM HERSTELLEN EINES SOLCHEN**
PROSTHESIS SOCKET AND METHOD FOR PRODUCING SAME
TIGE DE PROTHÈSE ET SON PROCÉDÉ DE FABRICATION

(30) Priorität: 18.12.2019 DE 102019134986
(43) Veröffentlichungstag der Anmeldung: 26.10.2022
(73) Patentinhaber: Ottobock SE & Co. KGaA, 37115 Duderstadt (DE)
(72) Erfinder: MAIER, Uli, 10405 Berlin (DE); VOLKMAR, Jens, 37115 Duderstadt (DE); LEINIGER, Andreas, 37327 Leinefeld (DE); VOORHEES, Coerte, 10405 Berlin (DE)
(74) Vertreter: Gramm, Lins & Partner Patent- und Rechtsanwälte PartGmbB
(86) Internationale Anmeldenummer: PCT/EP2020/086220
(87) Internationale Veröffentlichungsnummer: WO 2021/122597

(56) Entgegenhaltungen:
- EP-A1- 3 485 750
- WO-A1-2019/157486
- DE-A1- 102016 201 002
- US-B1- 6 436 149
- VITALI ANDREA ET AL: "Design and Additive Manufacturing of Lower Limb Prosthetic Socket", VOLUME 11: SYSTEMS, DESIGN, AND COMPLEXITY, 3 November 2017 (2017-11-03), pages 1 - 8, XP055782622, ISBN: 978-0-7918-5846-2, DOI: 10.1115/IMECE2017-71494
- POURFARZANEH AFONSO ET AL: "Towards Adaptive Prosthetic Sockets using 3D-printed Variable-stiffness Shape-memory Structures", 2019 2ND IEEE INTERNATIONAL CONFERENCE ON SOFT ROBOTICS (ROBOSOFT), IEEE, 14 April 2019 (2019-04-14), pages 410 - 415, XP033555796, DOI: 10.1109/ROBOSOFT.2019.8722811
- ROGERS BILL ET AL: "Advanced Trans-Tibial Socket Fabrication Using Selective Laser Sintering", PROSTHETICS AND ORTHOTICS INTERNATIONAL., vol. 31, no. 1, 1 March 2007 (2007-03-01), GB, pages 88 - 100, XP055782624, ISSN: 0309-3646, Retrieved from the Internet <URL:http://journals.sagepub.com/doi/full-xml/10.1080/03093640600983923> [retrieved on 20210305], DOI: 10.1080/03093640600983923

## Beschreibung

Die Erfindung betrifft ein Verfahren zum Herstellen eines Prothesenschaftes mit einem offenen proximalen Ende zum Aufnehmen eines Amputationsstumpfes, einer Innenwand und einer Außenwand. Prothesenschäfte dieser Art sind seit langem aus dem Stand der Technik bekannt und werden für unterschiedliche Prothesen und Amputationen verwendet. Sie dienen als Verbindungselement zwischen dem Amputationsstumpf des Patienten und der Prothese, die ein dem Patienten amputiertes Körperteil ersetzt. Insbesondere bei Beinprothesen sind die Prothesenschäfte einer großen mechanischen Belastung ausgesetzt. Dies gilt sowohl für einen Oberschenkelschaft als auch für einen Unterschenkelschaft.

Um ein angenehmes Tragegefühl und die benötigte Sicherheit dem Patienten zu vermitteln, ist es notwendig, dass der Schaft sicher am Amputationsstumpf angeordnet wird. Aus dem Stand der Technik sind eine Reihe unterschiedlicher Möglichkeiten bekannt, dies zu tun. Oftmals wird zunächst ein sogenannter Liner über den Amputationsstumpf gezogen, bevor die Kombination aus Amputationsstumpf und Liner in den Prothesenschaft eingesetzt wird. Dabei kann der Liner über einen Befestigungspin, der sich meist am distalen Ende des Liners befindet, mit einer dafür vorgesehenen Ausnehmung im Prothesenschaft zusammenwirken. Alternativ dazu wird zwischen dem Liner und dem Schaft ein Unterdruck in einem abgeschlossenen Volumen erzeugt, wodurch der Schaft am Liner und damit am Amputationsstumpf befestigt wird.

Der Träger des Prothesenschaftes, der die Prothese zum Gehen verwendet, muss die gesamte Prothese inklusive Schaft in der Schwungphase eines jeden Schrittes anheben und nach vorne schwingen. Dabei darf der Prothesenschaft dem Träger nicht das Gefühl vermitteln, die Verbindung zwischen Schaft und Amputationsstumpf sei unsicher. Gleichzeitig muss der Prothesenschaft an seinem distalen Ende ein Anschlusselement für weitere Prothesenelemente, beispielsweise ein Prothesenknie oder einen Unterschenkel mit einem daran angeordneten Knöchel und Fuß, aufweisen. Auch diese Verbindung, die über das Anschlusselement hergestellt wird, muss die beim Gehen auftretenden Kräfte sicher übertragen und dem Träger ein sicheres Tragegefühl vermitteln.

Die verschiedenen Elemente eines Prothesenschaftes sind daher relativ massiv ausgebildet, um den auftretenden Belastungen standhalten zu können. Dadurch wird insbesondere der Prothesenschaft schwer, wodurch der Tragekomfort reduziert wird. Moderne Prothesenschäfte werden oftmals mit Kohlefaser-Verbundwerkstoffen hergestellt. Diese sind leicht bei gleichzeitig großer mechanischer Stabilität und somit sehr gut geeignet, um die Anforderungen eines Prothesenschaftes zu erfüllen. In neuester Zeit werden Prothesenschäfte auch mittels eines additiven Fertigungsverfahrens, beispielsweise mittels eines 3D-Druckers hergestellt. Dadurch werden neue Ausgestaltungen und Formen des Prothesenschaftes möglich, die mit herkömmlichen Herstellungsmethoden, beispielsweise mit Kohlefaser-Verbundwerkstoffen, nicht herstellbar waren.

Aus der WO 2017/012888 A1 ist ein Herstellungsverfahren für einen Prothesenschaft bekannt, bei dem zunächst einen Innenschaft mittels eines 3D-Druckers hergestellt wird. Das verwendete Material ist dabei jedoch nicht in der Lage, die mechanischen Belastungen eines Prothesenschaftes zur Versorgung einer unteren Extremität aufzufangen und ihnen standzuhalten. Daher werden an besonders belasteten Stellen auf der Außenseite des so hergestellten Innenschaftes zusätzliche Verstärkungselemente aufgebracht, die dieses Problem lösen. Das Herstellverfahren wird dadurch jedoch aufwendiger, da mehrere Verfahrensschritte durchgeführt und mehrere unterschiedliche Materialien verwendet werden müssen.

Aus VITALI ANDREA ET AL: "Design and Additive Manufacturing of Lower Limb Prosthetic Socket",VOLUME 11: SYSTEMS, DESIGN, AND COMPLEXITY, 3. November 2017 (2017-11-03), Seiten 1-8, ist ein Herstellungsverfahren für einen Prothesenschaft bekannt, der ebenfalls eine Innenwand, eine Außenwand und dazwischen angeordnete Stützelemente aufweist. Die Positionen und Richtungen der Stützelemente werden jedoch nicht auf Grundlage der Richtungen der beim Gebrauch voraussichtlich auftretenden Kräfte bestimmt.

Der Erfindung liegt daher die Aufgabe zugrunde, die Nachteile aus dem Stand der Technik zu vermeiden oder zumindest zu reduzieren und ein Verfahren zur Herstellung eines Prothesenschaftes vorzuschlagen, der leicht ist und den Belastungen standhalten kann.

Die Erfindung löst die gestellte Aufgabe durch ein Verfahren zum Herstellen eines Prothesenschaftes gemäß Anspruch 1.

Durch die Ausgestaltung des Prothesenschaftes mit einem Hohlraum wird Gewicht reduziert, sodass der Prothesenschaft und damit die gesamte Prothese leichter ausgebildet ist. Gleichzeitig wird durch das interne Element, das sich durch den Hohlraum hindurch erstreckt, die Stabilität erhöht. Das interne Element ist einstückig mit der Innenwand ausgebildet und wird daher im gleichen Verfahrensschritt wie die jeweilige Wand hergestellt. Vorzugsweise ist die Innenwand, die Außenwand und das wenigstens eine interne Element einstückig miteinander ausgebildet.

Der Hohlraum befindet sich zwischen der Innenwand und der Außenwand und ist nicht der Raum, in den der Amputationsstumpf aufgenommen werden kann. Der Hohlraum ist vorzugsweise geschlossen ausgebildet, sodass keine Verbindung zwischen dem Inneren des Hohlraumes und der Umgebung besteht. Die Innenwand des Prothesenschaftes ist vorzugsweise der Form des aufzunehmenden Amputationsstumpfes zumindest grob nachgebildet. Sie ist im Wesentlichen becherförmig und der Prothesenschaft weist bevorzugt ein geschlossenes distales Ende auf. Der Hohlraum befindet sich vorzugsweise distal zum distalen Ende der Innenwand. Der wenigstens eine Hohlraum wird bevorzugt in Proximalrichtung durch die Innenwand des Prothesenschaftes und in allen anderen Richtungen durch die Außenwand des Prothesenschaftes begrenzt. Er kann, muss jedoch nicht rotationssymmetrisch um eine Längsachse ausgebildet sein. Am distalen Ende des Prothesenschaftes befindet sich vorzugsweise ein Anschlussmittel für ein weiteres distales Prothesenbauteil. Auch der Teil des Prothesenschaftes, an dem sich dieses Anschlussmittel befindet, ist Teil der Außenwand und begrenzt vorzugsweise den wenigstens einen Hohlraum in distaler Richtung. Bezüglich des Hohlraumes verfügen die Innenwand und die Außenwand über eine dem Hohlraum zugewandte Innenseite. An dieser Innenseite der jeweiligen Wand ist das wenigstens eine interne Element angeordnet.

Das wenigstens eine interne Element erstreckt sich durch den wenigstens einen Hohlraum. Es ragt also nicht nur in den wenigstens einen Hohlraum hinein, sondern durchläuft ihn vorzugsweise vollständig. Es verbindet vorzugsweise zwei Wände, die den wenigstens einen Hohlraum umgeben und ihn begrenzen, miteinander. Es kann sich zwischen der Innenwand und der Außenwand erstrecken oder mehrere Positionen oder Stellen einer einzigen Wand, also der Innenwand oder der Außenwand, miteinander verbinden.

Das wenigstens eine interne Element ist ein Stützelement, das sich bevorzugt von der Innenwand zu der Außenwand erstreckt. Ein solches Stützelement ist bevorzugt eine Strebe, ein Stab oder ein stangenförmiges Element, verfügt also in Längsrichtung, vorzugsweise also von der Innenwand zu der Außenwand, über eine größere Ausdehnung als in den anderen Raumrichtungen. Der Querschnitt kann kreisförmig, oval, viereckig, dreieckig, polygonal oder unregelmäßig sein. Ein Stützelement kann auch zwischen zwei Stellen oder Positionen einer einzigen Wand, also der Innenwand oder der Außenwand, angeordnet sein und diese Stellen oder Positionen miteinander verbinden. Der Querschnitt kann entlang des Verlaufes des Stützelementes variieren, insbesondere kann sich der Durchmesser ändern, so dass er beispielsweise im Bereich der Verbindung zwischen Wand und Stützelement größer ist. Selbstverständlich kann der Querschnitt auch über den Verlauf des Stützelementes konstant sein.

Besonders bevorzugt erstreckt sich das wenigstens eine Stützelement zumindest auch in proximal-distal-Richtung. Die Längsrichtung des wenigstens einen Stützelementes verfügt also zumindest auch über eine Komponente in dieser Richtung, wobei diese Komponente bevorzugt die größte Komponente ist. Das wenigstens eine Stützelemente erstreckt sich in diesem Fall also weiter in proximal-distal-Richtung als in alle anderen Richtungen.

Bevorzugt erstrecken sich in dem wenigstens einen Hohlraum wenigstens 25, bevorzugt wenigstens 100, besonders bevorzugt wenigstens 500 Stützelemente, von denen sich mehrere, bevorzugt alle, von der Innenwand zu der Außenwand erstrecken.

Dadurch wird eine gleichmäßige Lastverteilung auf die verschiedenen Stützelemente möglich und die Stabilität des Prothesenschaftes kann weiter optimiert werden.

In einer bevorzugten Ausgestaltung steht wenigstens ein Stützelement senkrecht auf der Innenwand und/oder der Außenwand. Besonders bevorzugt stehen mehrere, höchst bevorzugt alle, Stützelemente senkrecht auf der Innenwand und/oder der Außenwand. Dadurch werden Schermomente und Kippmomente auf die einzelnen Stützelemente vermieden, die ansonsten bei Belastung des Prothesenschaftes beispielsweise beim Gehen, auftreten könnten. Auch dies erhöht die Stabilität des Prothesenschaftes. Dadurch kann die Anzahl und die Dicke der Stützelemente reduziert und somit Material und Gewicht eingespart werden.

Vorzugsweise ist wenigstens ein internes Element ein Trennelement, das den Hohlraum in zwei Teil-Hohlräume unterteilt. Bevorzugt ist ein solches Trennelement ausschließlich an der Außenwand des Prothesenschaftes angeordnet. Im Gegensatz zu einem Stützelement ist ein Trennelement vorzugsweise flächig ausgebildet, verfügt also in zwei aufeinander senkrecht stehenden Richtungen über eine größere Ausdehnung als in der dritten Raumrichtung, die auf den beiden ersten Richtungen senkrecht steht. Das wenigstens eine Trennelement erstreckt sich vorzugsweise in einer Ebene, die im Wesentlichen senkrecht auf der proximal-distal-Richtung steht. In diesem Fall ist das wenigstens eine Trennelement vorzugsweise ausschließlich an der Innenseite der Außenwand angeordnet.

Bevorzugt verfügt das wenigstens eine Trennelement über wenigstens eine Öffnung, die die zwei Teil-Hohlräume miteinander verbindet. Besonders bevorzugt erstreckt sich durch diese Öffnung wenigstens ein Stützelement.

In einer alternativen Ausführungsform eines Trennelementes ist das Trennelement dreidimensional ausgebildet, verfügt also nicht über eine ausschließlich flächige Ausgestaltung. Es kann als hohle Röhre, gebogene Fläche oder in sonstiger Weise ausgebildet sein. Durch die Verwendung eines Trennelementes mit einer geschlossenen Querschnittskontur, beispielsweise einer Röhre wird der wenigstens eine Hohlraum in zwei Teil-Hohlräume unterteilt, von denen einer innerhalb und einer außerhalb des Trennelementes liegt. Die Querschnittskontur kann kreisförmig, oval, viereckig, dreieckig, polygonal oder unregelmäßig sein. Es ist auch möglich, dass die Kontur sich entlang einer Längsrichtung des so ausgebildeten Trennelementes in Form, Größe und/oder Kontur ändert. Ein in dieser Form ausgebildetes Trennelement erstreckt sich vorzugsweise im Wesentlichen entlang der proximal-distal-Richtung, so dass sich auch die durch das Trennelement erzeugten Teil-Hohlräume in dieser Richtung erstrecken. Das Trennelement ist bevorzugt zwischen der Innenwand im proximalen Bereich des Hohlraumes und der Außenwand im distalen Bereich des Hohlraumes angeordnet.

In einer bevorzugten Ausgestaltung verfügt der Prothesenschaft über eine Mehrzahl von Trennelementen, so dass der wenigstens eine Hohlraum in eine Mehrzahl von Teil-Hohlräumen unterteilt wird. Die Trennelemente verlaufen vorzugsweise parallel zueinander und besonders bevorzugt sind diese Trennelement flächig ausgebildet und verlaufen weiter bevorzugt senkrecht zur proximal-distal-Richtung. Es entstehen auf diese Weise Kammern, die eine Biegesteifigkeit des Prothesenschaftes in diesem Bereich deutlich erhöhen.

Bevorzugt ist an einem distalen Ende des Prothesenschaftes wenigstens ein Anschlussmittel für ein distales Prothesenbauteil angeordnet. Das Anschlussmittel ist bevorzugt an der Außenwand des Prothesenschaftes angeordnet. Das distale Prothesenbauteil ist beispielsweise ein Prothesenknie oder ein Unterschenkelrohr oder ein Prothesenfuß vorzugsweise mit einem künstlichen Knöchelgelenk. Insbesondere, aber nicht nur bei Unterschenkelprothesenschäften, ist es von Vorteil, eine Ausdehnung des Prothesenschaftes in distaler Richtung so weit zu vergrößern, den Prothesenschaft also in dieser Richtung so weit zu verlängern, dass direkt ein Prothesenfuß und/oder ein künstliches Knöchelgelenk an dem Anschlussmittel positioniert werden kann. Dadurch wird die Anzahl der benötigen Prothesenbauteile reduziert und die Montage der Prothese vereinfacht. Es kann beispielsweise auf ein separates Unterschenkelrohr verzichtet werden. In dem so verlängerten Bereich des Prothesenschaftes befindet sich zumindest auch der wenigstens eine Hohlraum, der weiter bevorzugt durch wenigstens ein, vorzugsweise jedoch mehrere, Trennelemente in mehrere Teil-Hohlräume oder Kammern unterteilt ist.

Verfügt der Prothesenschaft über mehrere Stützelemente, verlaufen gedachte Verlängerungen der Stützelemente durch das Anschlussmittel. Besonders bevorzugt treffen sie sich in einem einzigen Punkt innerhalb des Anschlussmittels. Die Kraftübertragung durch die Stützelemente erfolgt in diesem Fall direkt in das Anschlussmittel und damit direkt in ein an dem Anschlussmittel angeordnetes Prothesenbauteil. Auch dadurch können Schermomente oder Kippmomente vermieden oder zumindest verringert werden.

Bevorzugt handelt es sich bei dem Prothesenschaf um einen Prothesenschaft für eine Prothese zur Versorgung der unteren Extremität, bevorzugt für eine Unterschenkelprothese.

Ein Prothesenschaft der hier beschriebenen Art verfügt vorzugsweise über wenigstens ein erstes Einlegeelement, das eine Anlagefläche aufweist, die zumindest zum teil, bevorzugt jedoch vollständig korrespondierend zu wenigstens einem Teil der Innenwand des Prothesenschaftes ausgebildet ist. Ein derartiges Einlegeelement ist auch bei anderen Prothesenschäften, die beispielsweise keinen Hohlraum und/oder kein internes Element aufweisen, von Vorteil. Daher stellt ein solches Einlegeelement in Kombination mit den Merkmalen des Oberbegriffs des ursprünglichen Anspruchs 1 eine eigene Erfindung dar, die mit oder ohne die Merkmale der hier beschriebenen Prothesenschäfte verwendbar ist. Ein Einlegeelement wird verwendet, wenn der Amputationsstumpf im Laufe der Zeit Volumen verliert und der ursprüngliche Prothesenschaft nicht mehr optimal an den Amputationsstumpf angepasst ist. Dann wird ein Einlegeelement hergestellt, das mühsam an die gewünschte Form angepasst, beispielsweise geschliffen, werden muss. Dies ist aufwendig und daher kostenintensiv.

Mit der vorliegenden Erfindung lässt sich dies vereinfachen. Der Prothesenschaft wird vorzugsweise in einem additiven Fertigungsverfahren, beispielsweise einem 3D-Druck-Verfahren hergestellt, so dass seine Konturen und Formen elektronisch gespeichert vorliegen. Daher kann einfach ein erstes Einlegeelement hergestellt, insbesondere ebenfalls 3D-gedruckt werden, dessen Anlagefläche optimal an die Innenwand des Prothesenschaftes, bevorzugt deren der Innenseite gegenüberliegende Außenseite, angepasst ist. Bevorzugt kann das Einlegeelement vollflächig an die Außenseite der Innenwand des Prothesenschaftes angelegt werden. Das Einlegeelement wird also in den Hohlraum, der zur Aufnahme des Amputationsstumpfes vorgesehen ist, eingebracht. Ein aufwändiges Nacharbeiten ist nicht nötig. In der gewünschten Position wird das Einlegeelement mit der Innenwand des Prothesenschaftes verklebt oder auf sonstige Weise fixiert. Vorzugsweise bildet das Einlegeelement dann mit dem nicht von ihm abgedeckten Teil der Außenseite der Innenwand der Prothesenschaftes eine einheitliche und insbesondere stetige, also stufenlose, Oberfläche.

Vorzugsweise verfügt der Prothesenschaft über wenigstens ein zweites Einlegeelement, das eine Anlagefläche aufweist, die korrespondierend zu der Innenwand des Prothesenschaftes und/oder korrespondierend einer der Anlagefläche gegenüberliegenden Fläche des ersten Einlegeelementes ausgebildet ist. Der Prothesenschaft wird bevorzugt mit mehreren derartiger Einlegeelemente ausgeliefert und bildet mit diesen vorzugsweise ein Set. Alternativ oder zusätzlich dazu können auch Einlegeelemente mit der passenden Materialstärke individuell angefertigt werden. Kommt es zu einer dauerhaften Volumenänderung, insbesondere einem Volumenrückgang, des Amputationsstumpfes, kann diese durch geeignete Auswahl eines oder mehrerer Einlegeelemente ausgeglichen werden.

Um die Richtungen der im Gebrauch voraussichtlich auftretenden Kräfte bestimmen zu können, wird vorzugsweise ein Modell der herzustellenden Prothese erstellt, das in einer elektronischen Datenverarbeitungseinrichtung bearbeitbar ist. Es ist vorzugsweise in einem elektronischen Datenspeicher hinterlegt. Eine Simulationseinheit der elektronischen Datenverarbeitungseinrichtung simuliert dann mit Hilfe eines ebenfalls hinterlegten Modells die auftretenden Kräfte und speichert ihre Beträge und Richtungen ab. Eine Modellierungseinheit der elektronischen Datenverarbeitungseinrichtung modelliert auf der Grundlage dieser Daten die optimalen Positionen und Orientierungen der benötigten Stützelemente. Zusätzlich können die Dicke, der Querschnitt, der Verlauf und/oder die Anzahl der Stützelemente als Optimierungsparameter von der

Modellierungseinheit verwendet werden. Die elektronische Datenverarbeitungseinrichtung übermittelt dann Steuerbefehle an die additive Fertigungsanlage, beispielsweise den 3D-Drucker und steuert diese derart an, dass der modellierte Prothesenschaft gedruckt wird.

Bevorzugt wird der Prothesenschaft zumindest teilweise mittels additiver Fertigungsverfahren, beispielsweise mittels eines 3D-Druckers hergestellt, wobei insbesondere das wenigstens eine Stützelement additiv hergestellt, beispielsweise gedruckt wird.

Mithilfe der beiliegenden Figuren werden nachfolgend einige Ausführungsbeispiele der vorliegenden Erfindung näher erläutert.

Es zeigen
- Figur 1 -: die schematische Darstellung einer Unterschenkelprothese,
- Figur 2 -: die Darstellung aus Figur 1 mit dem Prothesenschaft in einer Schnittdarstellung,
- Figuren 3-6: schematische Schnittdarstellungen durch Prothesenschäfte,
- Figuren 7-8: schematische Darstellungen von Einlegeelement,
- Figur 9 -: schematische Schnittdarstellungen durch den Hohlraum,
- Figur 10 -: schematische Darstellungen eines Anschlussmittels
- Figur 11 -: die schematische Schnittdarstellung durch einen Schaft mit Anschlussmittel,
- Figur 12 -: die schematische Darstellung eines Teils eines Prothesenschaftes,
- Figur 13 -: die schematische Schnittdarstellung durch einen Teil eines Prothesenschaftes mit Anschlussmittel und
- Figur 14 -: eine schematische Schnittdarstellung entlang einer anderen Ebene durch einen Prothesenschaft.

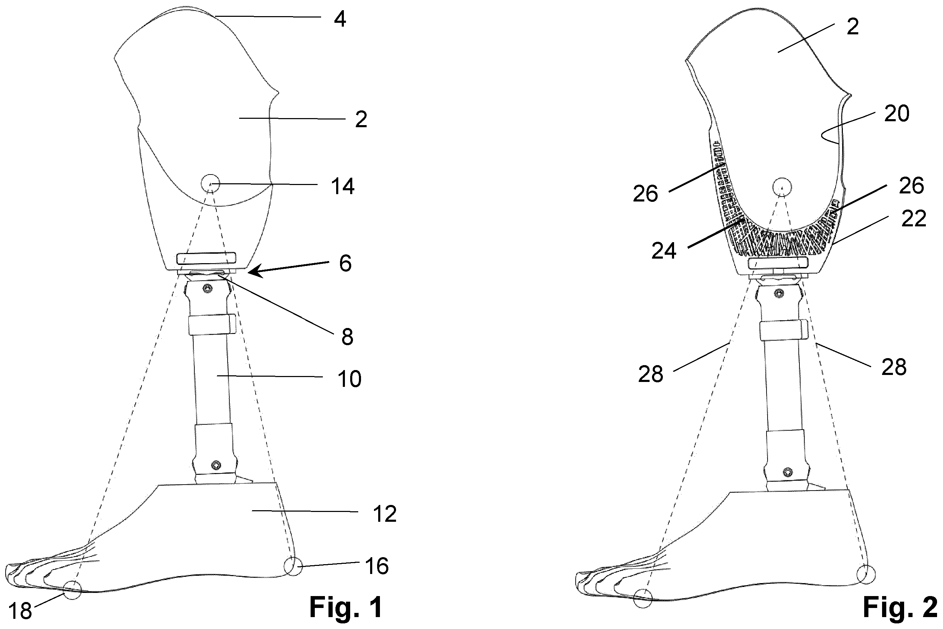

Figur 1 zeigt die schematische Darstellung einer Unterschenkelprothese, die einen Prothesenschaft 2 aufweist, der ein offenes proximales Ende 4 und ein geschlossenes distales Ende 6 aufweist. An dem distalen Ende 6 befindet sich ein Anschlussmittel 8, an dem ein Unterschenkelrohr 10 angeordnet ist. Am unteren Ende dieses Unterschenkelrohres 10 befindet sich ein Prothesenfuß 12. Für die Bestimmung von Position und Orientierung wurde im gezeigten Ausführungsbeispiel eine virtuelle Schwenkachse 14 für ein in dem Prothesenschaft 2 aufgenommenes Knie angenommen. Diese Schwenkachse 14 wurde einerseits mit einer Fersenauftrittsstelle 16 und andererseits mit einer Stelle 18 verbunden, bei der die Belastung beim Übergang in die Schwungphase anliegt. Es ergibt sich der maximale Belastungswinkel.

Figur 2 zeigt die Darstellung aus Figur 1 bei der nun der Prothesenschaft 2 in einer Schnittdarstellung dargestellt ist. Er verfügt über eine Innenwand 20 und eine Außenwand 22, zwischen denen sich ein Hohlraum 24 befindet. In diesem Hohlraum 24 sind im gezeigten Ausführungsbeispiel eine Vielzahl von Stützelementen 26 angeordnet, die sich alle durch den Hohlraum 24 erstrecken. Die meisten sind mit einem Ende an einer Innenseite der Innenwand 20 und mit dem gegenüberliegenden Ende an der Innenseite der Außenwand 22 angeordnet. Einige wenige erstrecken sich jedoch zwischen zwei unterschiedlichen Stellen der Innenseite der Außenwand 22. Man erkennt anhand der gestrichelten Linien 28, dass einige der Stützelemente 26 entlang dieser Linien 28 verlaufen und so beim Fersenauftritt und beim Abheben der Zehen die optimale Kraftübertragung ermöglichen. Die übrigen Stützelemente 26 sind auf ähnliche Weise konfiguriert worden. Für unterschiedliche Belastungen bei unterschiedlichen Bewegungen und/oder in unterschiedlichen Phasen eines Gangzyklus werden unterschiedliche Stützelemente maximal belastet. Diese erstrecken sich zum jeweiligen Zeitpunkt in optimaler Richtung für die Kraftübertragung.

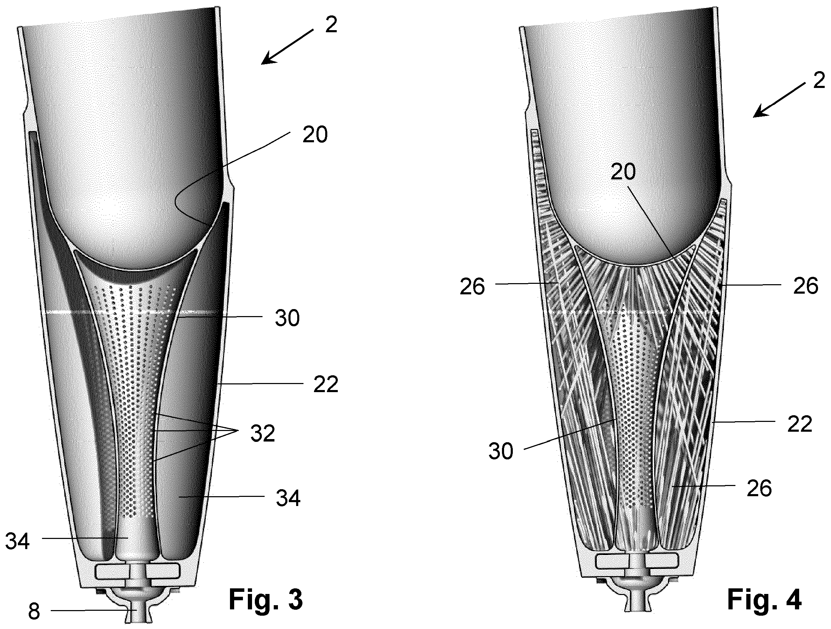

Figur 3 zeigt eine schematische Schnittdarstellung durch einen anderen Prothesenschaft 2. Auch er verfügt über eine Innenwand 20 und eine Außenwand 22 zwischen denen sich ein Hohlraum 24 befindet. Innerhalb des Hohlraums ist ein Trennelement 30 vorhanden, das im gezeigten Ausführungsbeispiel eine geschlossene Querschnittskontur aufweist. Das Trennelement 30 verfügt über eine Vielzahl von Öffnungen 32, die als kleine Punkte dargestellt sind. Das Trennelement 30 erstreckt sich von der Innenwand 20 im proximalen Bereich des Hohlraums 24 bis zum distalen Ende des Hohlraums 24 und endet dort an der Außenwand 22. Es entstehen dadurch zwei Teil-Hohlräume 34, von denen sich einer außerhalb des Trennelementes 30 zwischen dem Trennelement 30 und der Außenwand 22 befindet. Der andere Teil-Hohlraum 24 befindet sich innerhalb des Trennelementes 30. Man erkennt in Figur 3, dass der Prothesenschaft 2 in distaler Richtung verlängert wurde, sodass das Anschlussmittel 8, das im gezeigten Ausführungsbeispiel als Pyramiden-Adapter ausgebildet ist, weiter in distaler Richtung verschoben wird. Wird der in Figur 3 schematisch dargestellte Prothesenschaft 2 für eine Unterschenkelprothese verwendet, wie sie beispielsweise in den Figuren 1 und 2 dargestellt ist, ist es nicht mehr notwendig, ein separates Unterschenkelrohr 10 zu verwenden. Der verlängerte distale Bereich des Prothesenschaftes 2 übernimmt diese Aufgaben und ist aufgrund der Strukturen innerhalb des Hohlraums 24 in der Lage, den auftretenden Belastungen standzuhalten.

Figur 4 zeigt eine weitere Darstellung eines Prothesenschaftes 2 mit der Innenwand 20, der Außenwand 22 und des in Figur 3 bereits gezeigten Trennelementes 30. Zusätzlich verfügt der Prothesenschaft 2 in Figur 4 über eine Vielzahl von Stützelementen 26. Von diesen Stützelementen 26 erstrecken sich einige von der Innenwand 20 zur Außenwand 22, wobei einige dieser Stützelemente 26 durch die Öffnungen 32 im Trennelement 30 hindurch geführt sind. Einige der Stützelemente 26 verlaufen vollständig im äußeren Teil-Hohlraum 34 und verlaufen von einer ersten Stelle der Außenwand 22 zu einer zweiten Stelle der Außenwand 22. Die meisten dieser Stützelemente 26 erstrecken sich zumindest vorwiegend in proximal-distal-Richtung.

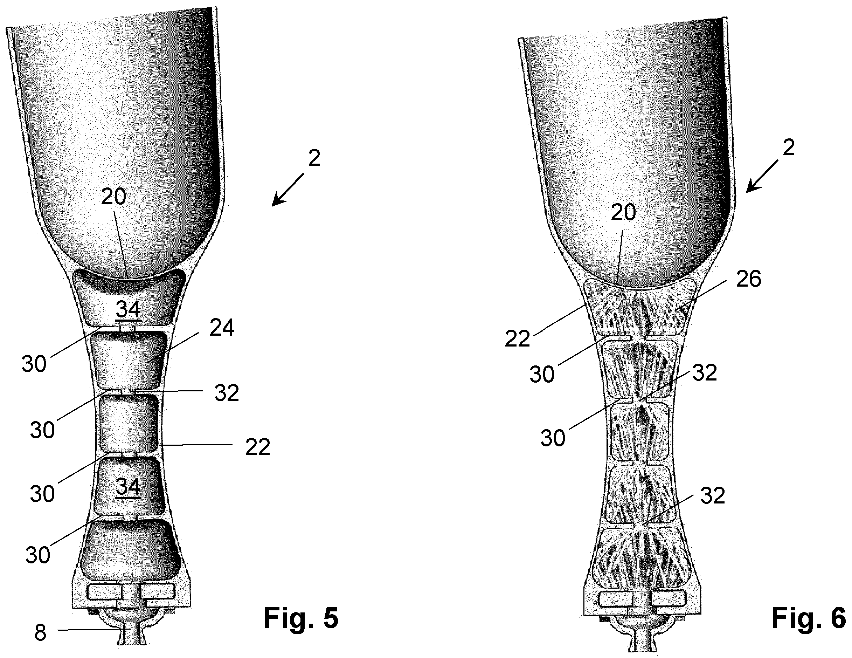

Figur 5 zeigt eine weitere schematische Darstellung eines Prothesenschaftes 2 in einer Schnittdarstellung. Auch dieser Prothesenschaft 2 verfügt über die Innenwand 20, die Außenwand 22 sowie das Anschlussmittel 8 für ein distales Prothesenelement. Auch bei dem in Figur 5 gezeigten Prothesenschaft 2 ist der distale Bereich verlängert, um so beispielsweise ein Unterschenkelrohr überflüssig zu machen. Zwischen der Innenwand 20 und der Außenwand 22 befindet sich der Hohlraum 24, der im gezeigten Ausführungsbeispiel durch vier Trennelemente 30 in fünf Teil-Hohlräume 34 unterteilt ist. Die Trennelemente 30 verlaufen parallel zueinander und sind flächig ausgebildet. Sie verfügen anders als das Trennelement 30 aus Figur 3 und 4 nicht über eine geschlossene Querschnittskontur. Die Trennelemente 30 in Figur 5 verfügen über jeweils eine Öffnung 32, durch die die jeweils angrenzenden Teil-Hohlräume 34 miteinander verbunden sind.

Figur 6 zeigt in einer weiteren Schnittdarstellung einen Prothesenschaft 2, der ähnlich dem in Figur 5 gezeigten Prothesenschaft 2 aufgebaut ist. Auch er verfügt über vier Trennelemente 30, die den distalen Bereich des Prothesenschaftes in Teil-Hohlräume 34 aufteilen. Zusätzlich verfügt der in Figur 6 gezeigte Prothesenschaft 2 über eine Vielzahl von Stützelementen 26, die teilweise durch die Öffnungen 32 in den Trennelement 30 hindurch verlaufen. Andere erstrecken sich zwischen 2 Trennelement 30 oder verlaufen von der Innenwand 20 zur Außenwand 22.

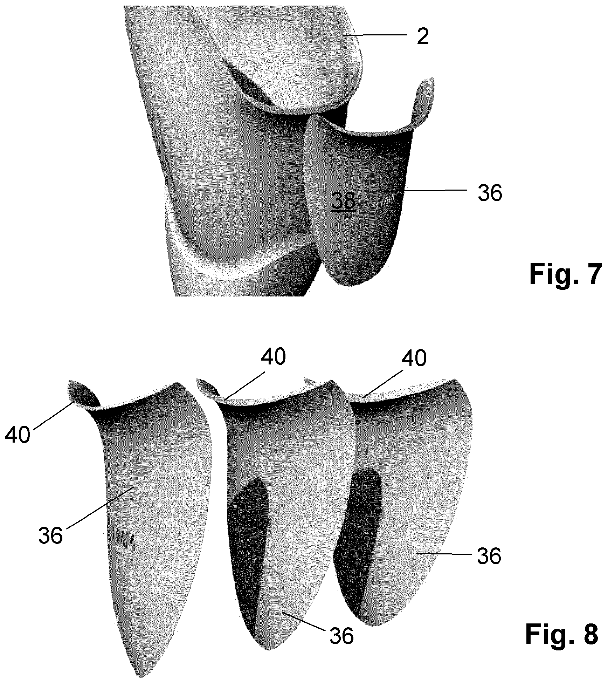

Figur 7 zeigt schematisch einen Prothesenschaft 2 mit einem für diesen Prothesenschaft 2 gefertigten Einlegeelement 36 es verfügt über eine Anlagefläche 38, die zu einer Außenseite der Innenwand 20 des Prothesenschaftes 2 korrespondierend ausgebildet ist, sodass das Einlegeelement 36 mit der Anlagefläche 38 vollflächig an der Innenwand 20 des Prothesenschaftes 2 angeordnet werden kann. Auf diese Weise wird einer Volumenreduzierung des Amputationsstumpfes, der in den Prothesenschaft 2 aufgenommen werden soll, Rechnung getragen.

Figur 8 zeigt drei verschiedene Einlegeelement 36, die über unterschiedliche Dicken verfügen. Dies ist insbesondere an der Krempe 40 gut zu erkennen. Je nach Stärke der Volumenreduzierung des Amputationsstumpfes kann auf diese Weise das jeweils passende Einlegeelement 36 verwendet werden. Die Einlegeelement 36 sind vorzugsweise 3D gedruckt und können auf diese Weise besonders einfach an die Innenwand 20 des Prothesenschaftes angepasst und zu ihr korrespondierend ausgebildet werden.

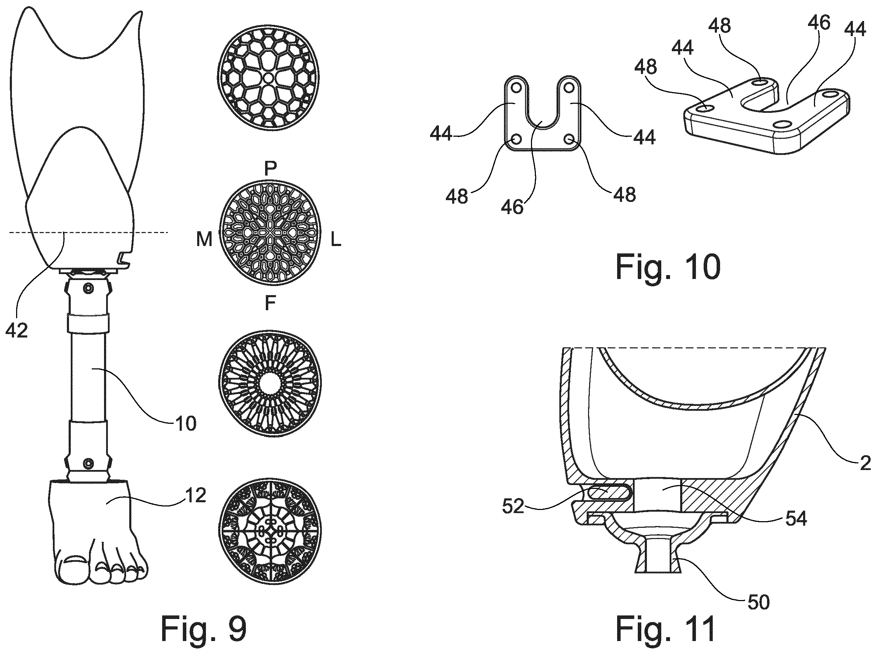

Figur 9 zeigt im linken Teil schematisch eine Frontalansicht eine Unterschenkelprothese mit dem Prothesenschaft 2, dem Unterschenkelrohr 10 und dem Prothesenfuß 12. Im rechten Teil der Figur 9 sind übereinander vier Schnittdarstellungen durch den Prothesenschaft 2 entlang der gestrichelten Linien 42 dargestellt. Man erkennt zum Teil komplexe Muster aus Stützelementen 26 und Trennelement 30, wobei diese in den gezeigten Darstellungen nicht zu unterscheiden sind, da nicht klar zu erkennen ist, zwischen welchen zwei Punkten sich die internen Elemente erstrecken. Bei den vier übereinander dargestellten Schnittdarstellungen liegt die mediale Seite links, die lateraler Seite rechts und die frontale Seite unten.

Figur 10 zeigt schematisch die Darstellung eines Anschlussmittels 8 in zwei unterschiedlichen Perspektiven. Es verfügt über eine im Wesentlichen u-förmige Gestalt mit zwei Schenkeln 44 und einer dazwischenliegenden Ausnehmung 46. In der gezeigten Ausführungsform verfügt das Anschlussmittel 8 über vier Bohrlöcher 48, die bevorzugt mit einem Innengewinde versehen sind. Auf diese Weise lassen sich Schrauben in ihnen befestigen, um so ein weiteres Anschlussmittel, beispielsweise einen Pyramidenadapter 50 zu befestigen.

Dies ist in Figur 11 dargestellt. Im distalen Bereich des gezeigten Prothesenschaftes 2 befindet sich eine schlitzförmige Vertiefung 52, die vorzugsweise einstückig mit dem Rest des Prothesenschaftes 2 ausgebildet ist. Sie wird also bevorzugt nicht nach der Fertigung des Prothesenschaftes 2 beispielsweise mittels einer Fräse eingebracht, sondern vorzugsweise in einem additiven Fertigungsverfahren gemeinsam mit dem Rest des Prothesenschaftes 2 einstückig ausgebildet. Das in Figur 10 gezeigte Anschlussmittel 8 kann in diese Vertiefung eingebracht werden, sodass der dargestellt Pyramidenadapter 50 angeschraubt werden kann. Ein die großen Belastungen übertragender Stamm 54 wird dabei in der Ausnehmung 46 im Anschlussmittel 8 angeordnet, sodass die teilweise großen mechanischen Kräfte, die insbesondere bei Beinprothesen auftreten können, übertragen werden.

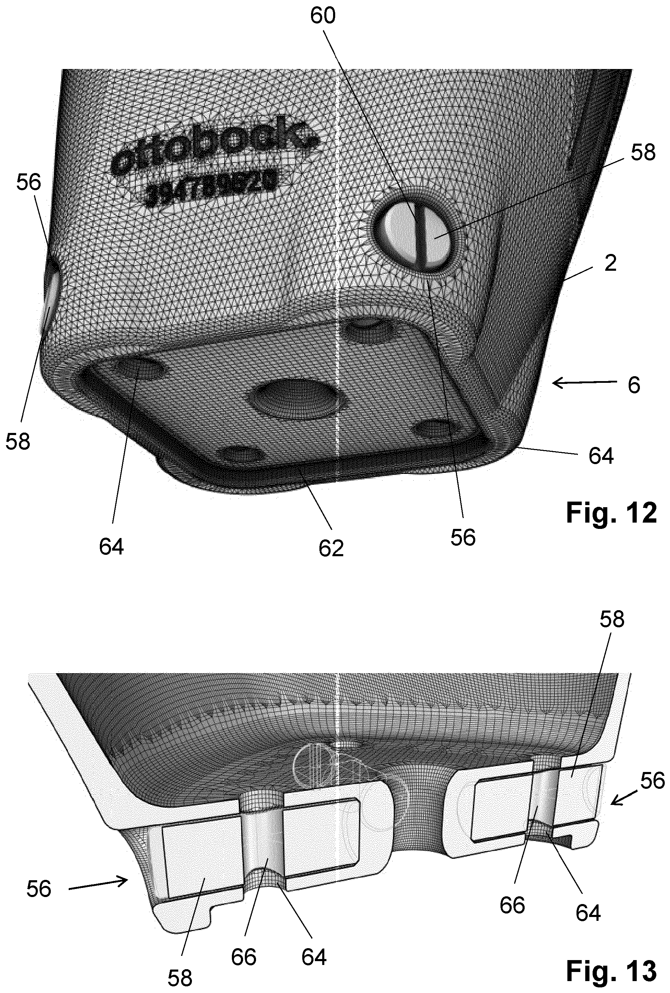

Figur 12 zeigt einen Ausschnitt eines Prothesenschaftes 2 und insbesondere dessen distales Ende 6. Er verfügt über vier Ausnehmungen 56, die in den Eckbereichen angeordnet sind und von denen in Figur 12 zwei zu erkennen sind. Darin sind in Form von Bolzen 58 die Anschlussmittel 8 enthalten. Im gezeigten Ausführungsbeispiel verfügen die Bolzen 58 an ihrer nach außenweisenden Seite über einen Schlitz 60, sodass sie mittels eines geeigneten Werkzeuges, beispielsweise eines Schraubendrehers, bewegt werden können. An einer distalen Seite 62 des Prothesenschaftes 2 befinden sich vier Löcher 64, in die jeweils eine Schraube eingeführt werden kann, um beispielsweise einen in Figur 12 nicht gezeigten Pyramidenadapter 50 zu befestigen. Besonders vorteilhaft verfügen die Bolzen 58 über ein entsprechen des Gewinde, in das die Schrauben eingeschraubt werden können, wenn der Bolzen 58 in der richtigen Position und Orientierung in der Ausnehmung 56 angeordnet ist.

Figur 13 zeigt die Situation in einer Schnittdarstellung. Man erkennt zwei Bolzen 58, die in jeweils eine Ausnehmung 56 eingeführt worden sind. Sie verfügen jeweils über eine Bohrung 66, die vorzugsweise mit dem Gewinde ausgebildet ist, dass in Figur 13 jedoch nicht dargestellt ist. Durch die Löcher 64 in der distalen Seite 62 des Prothesenschaftes 2 können die Schrauben eingeführt werden, die vorzugsweise mit dem an der Innenwand der Bohrung 66 vorhandenen Gewinde zusammenwirken und so einen in Figur 13 nicht dargestellten Adapter, beispielsweise einen Pyramidenadapter 50 zu befestigen.

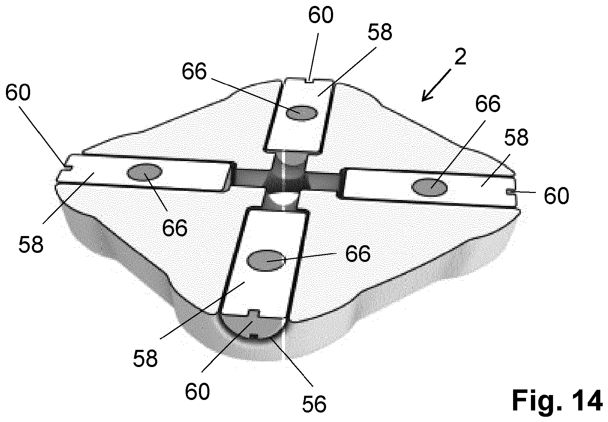

Figur 14 zeigt eine weitere Schnittdarstellung durch den Prothesenschaft 2 parallel zur distalen Seite 62 des Prothesenschaftes 2. Die vier Bolzen 58 sind jeweils in eine der Ausnehmungen 56 eingeführt und verfügen wie bereits dargelegt über den Schlitz 60, sodass sich ihre Ausrichtung und Orientierung relativ zum Rest des Prothesenschaftes 2 und insbesondere relativ zu den Löchern 64 an der distalen Seite 62 des Prothesenschaftes 2 eingestellt werden kann. Vorzugsweise ist die gewünschte Position sehr leicht zu erreichen, beispielsweise in dem der Schlitz 60 wie in Figur 14 dargestellt senkrecht angeordnet wird. Jeder Bolzen 58 verfügt über die Bohrung 66, die durch Rotation der Bolzen 58 um ihre Längsachse in Überdeckung mit den in Figur 14 nicht dargestellten Löchern 64 an der distalen Seite 62 des Prothesenschaftes 2 gebracht werden können, sodass die nicht dargestellten Schrauben zum Befestigen eines Adapter, insbesondere eines Pyramidenadapter 50 eingeschraubt werden können.

### Bezugszeichenliste:

- 2: Prothesenschaft
- 4: proximales Ende
- 6: distales Ende
- 8: Anschlussmittel
- 10: Unterschenkelrohr
- 12: Prothesenfuß
- 14: virtuelle Schwenkachse
- 16: Fersenauftrittsstelle
- 18: Stelle
- 20: Innenwand22 Außenwand
- 24: Hohlraum
- 26: Stützelement
- 28: gestrichelte Linie
- 30: Trennelement
- 32: Öffnung
- 34: Teil-Hohlraum
- 36: Einlegeelement
- 38: Anlagefläche
- 40: Krempe
- 42: gestrichelte Linie
- 44: Schenkel
- 46: Ausnehmung
- 48: Bohrloch
- 50: Pyramidenadapter
- 52: Vertiefung
- 54: Stamm
- 56: Ausnehmung
- 58: Bolzen
- 60: Schlitz
- 62: distale Seite
- 64: Loch
- 66: Bohrung

## Patentansprüche

1. Verfahren zum Herstellen eines Prothesenschaftes (2) mit
- einem offenen proximalen Ende (4) zum Aufnehmen eines Amputationsstumpfes,
- einer Innenwand und
- einer Außenwand,
wobei zwischen der Innenwand und der Außenwand wenigstens ein Hohlraum (24) angeordnet ist, durch den sich wenigstens ein internes Element erstreckt, wobei das wenigstens eine interne Element einstückig mit der Innenwand und/oder der Außenwand ausgebildet ist, wobei wenigstens ein internes Element ein Stützelement (26) ist,
wobei bei dem Verfahren
- die Richtungen der beim Gebrauch des Prothesenschaftes voraussichtlich auftretenden Kräfte ermittelt werden,
- auf der Grundlage dieser Richtungen die Positionen und Richtungen des wenigstens einen Stützelementes (26) bestimmt werden und
auf der Grundlage dieser Positionen und Richtungen der Stützelemente (26) der Prothesenschaft (2) gefertigt wird.

2. Verfahren nach Anspruch 1, **dadurch gekennzeichnet, dass** der Prothesenschaft (2) zumindest teilweise mittels eines additiven Fertigungsverfahrens hergestellt wird, wobei insbesondere das wenigstens eine Stützelement (26) in dem additiven Fertigungsverfahren hergestellt wird.

3. Verfahren nach Anspruch 1 oder 2, **dadurch gekennzeichnet, dass** sich das Stützelement (26) von der Innenwand zu der Außenwand erstreckt.

4. Verfahren nach einem der vorstehenden Ansprüche, **dadurch gekennzeichnet, dass** sich in dem wenigstens einen Hohlraum (24) wenigstens 25, bevorzugt wenigstens 100, besonders bevorzugt wenigstens 500 Stützelemente (26) von der Innenwand zu der Außenwand erstrecken.

5. Verfahren nach einem der vorstehenden Ansprüche, **dadurch gekennzeichnet, dass** wenigstens ein Stützelement (26), bevorzugt eine Mehrzahl von Stützelementen (26), senkrecht auf der Innenwand und/oder senkrecht auf der Außenwand steht.

6. Verfahren nach einem der vorstehenden Ansprüche, **dadurch gekennzeichnet, dass** wenigstens ein internes Element ein Trennelement (30) ist, das den Hohlraum (24) in zwei Teil-Hohlräume (34) unterteilt und bevorzugt ausschließlich an der Außenwand angeordnet ist.

7. Verfahren nach Anspruch 6, **dadurch gekennzeichnet, dass** das wenigstens eine Trennelement (30) wenigstens eine Öffnung aufweist, die die zwei Teil-Hohlräume (34) verbindet.

8. Verfahren nach Anspruch 7, **dadurch gekennzeichnet, dass** wenigstens ein Stützelement (26) durch die wenigstens eine Öffnung des wenigstens einen Trennelementes (30) verläuft.

9. Verfahren nach einem der Ansprüche 6 bis 8, **dadurch gekennzeichnet, dass** eine Mehrzahl von internen Elementen Trennelemente (30) sind, die bevorzugt parallel zueinander verlaufen.

10. Verfahren nach einem der vorstehenden Ansprüche, **dadurch gekennzeichnet, dass** an dem distalen Ende (6) des Prothesenschaftes (2) ein Anschlussmittel (8) für ein distales Prothesenbauteil angeordnet ist und sich gedachte Verlängerungen einer Mehrzahl von Stützelementen (26) in dem Anschlussmittel (8), bevorzugt in einem Punkt innerhalb des Anschlussmittels (8) treffen.

11. Verfahren nach einem der vorstehenden Ansprüche, **dadurch gekennzeichnet, dass** der Prothesenschaft (2) ein Prothesenschaft für eine Prothese zur Versorgung einer unteren Extremität, bevorzugt für eine Unterschenkelprothese, ist.

12. Verfahren nach einem der vorstehenden Ansprüche, **dadurch gekennzeichnet, dass** der Prothesenschaft (2) wenigstens ein erstes Einlegeelement (36) aufweist, das eine Anlagefläche (38) aufweist, die korrespondierend zu der Innenwand des Prothesenschaftes ausgebildet ist.

13. Verfahren nach Anspruch 12, **dadurch gekennzeichnet, dass** der Prothesenschaft (2) wenigstens ein zweites Einlegeelement (36) aufweist, das eine Anlagefläche (38) aufweist, die korrespondierend zu der Innenwand des Prothesenschaftes und/oder korrespondierend einer der Anlagefläche (38) gegenüberliegenden Fläche des ersten Einlegeelementes (36) ausgebildet ist.

## Claims

1. A method for producing a prosthesis socket (2) with
- an open proximal end (4) for accommodating an amputation stump,
- an inner wall and
- an outer wall,
wherein
at least one cavity (24) is arranged between the inner wall and the outer wall, through which at least one internal element extends, the at least one internal element being designed as a single piece with the inner wall and/or outer wall,
wherein at least one internal element is a support element (26), and wherein in said method
- the directions of the forces expected to occur during use of the prosthesis socket are determined,
- on the basis of these directions, the positions and directions of the at least one support element (26) are determined, and
- on the basis of these positions and directions of the support elements (26) the prosthesis socket (2) is produced.

2. The method according to claim 1, **characterized in that** the prosthesis socket (2) is at least partially produced by means of an additive manufacturing process, wherein the at least one support element (26) in particular is produced in the additive manufacturing process.

3. The method according to claim 1 or 2, **characterized in that** the support element (26) extends from the inner wall to the outer wall.

4. The method according to one of the preceding claims, **characterized in that** at least 25, preferably at least 100, especially preferably at least 500 support elements extend in the at least one cavity from the inner wall to the outer wall.

5. The method according to one of the preceding claims, **characterized in that** at least one support element (26), preferably a plurality of support elements (26), is perpendicular to the inner wall and/or perpendicular to the outer wall.

6. The method according to one of the preceding claims, **characterized in that** the at least one internal element is a partition element (30) that divides the cavity (24) into two partial cavities (34) and is preferably arranged only on the outer wall.

7. The method according to claim 6, **characterized in that** the at least one partition element (30) has at least one opening that connects the two partial cavities (34).

8. The method according to claim 7, **characterized in that** at least one support element (26) extends through the at least one opening of the at least one partition element (30).

9. The method according to one of the claims 6 to 8, **characterized in that** a plurality of internal elements are partition elements (30) that preferably run parallel to each other.

10. The method according to one of the preceding claims, **characterized in that** a connection means (8) for a distal prosthesis component is arranged on the distal end (6) of the prosthesis socket (2) and imaginary extensions of a plurality of support elements (26) in the connection means (8) preferably meet at one point within the connection means (8).

11. The method according to one of the preceding claims, **characterized in that** the prosthesis socket (2) is a prosthesis socket for a prosthesis for treating a lower limb, preferably a lower leg prosthesis.

12. The method according to one of the preceding claims, **characterized in that** the prosthesis socket (2) comprises at least a first insert element (36) that has a contact surface (38) which is designed to correspond to the inner wall of the prosthesis socket.

13. The method according to claim 12, **characterized in that** the prosthesis socket (2) has at least a second insert element (36) that features a contact surface (38), which is designed to correspond to the inner wall of the prosthesis socket and/or a surface of the first insert element (36) that is opposite the contact surface (38).

## Revendications

1. Procédé de réalisation d'une emboîture de prothèse (2) comprenant
- une extrémité proximale ouverte (4) destinée à recevoir un moignon d'amputation,
- une paroi intérieure, et
- une paroi extérieure,
au moins une cavité (24) étant disposée entre la paroi intérieure et la paroi extérieure, à travers laquelle s'étend au moins un élément interne, ledit au moins un élément interne étant formé d'un seul tenant avec la paroi intérieure et/ou avec la paroi extérieure, au moins un élément interne étant un élément de soutien (26),
procédé dans lequel
- on détermine les directions des forces qui se produisent probablement pendant l'utilisation de l'emboîture de prothèse,
- en se basant sur ces directions, on définit les positions et les directions dudit au moins un élément de soutien (26), et
en se basant sur ces positions et directions des éléments de soutien (26), on réalise l'emboîture de prothèse (2).

2. Procédé selon la revendication 1,
**caractérisé en ce que** l'emboîture de prothèse (2) est réalisée au moins en partie par un procédé de fabrication additive, en particulier, ledit au moins un élément de soutien (26) étant réalisé dans le cadre du procédé de fabrication additive.

3. Procédé selon la revendication 1 ou 2,
**caractérisé en ce que** l'élément de soutien (26) s'étend de la paroi intérieure vers la paroi extérieure.

4. Procédé selon l'une des revendications précédentes,
**caractérisé en ce que** dans ladite au moins une cavité (24), au moins 25, de préférence au moins 100, de préférence encore au moins 500 éléments de soutien (26) s'étendent de la paroi intérieure vers la paroi extérieure.

5. Procédé selon l'une des revendications précédentes,
**caractérisé en ce qu'**au moins un élément de soutien (26), de préférence une pluralité d'éléments de soutien (26), est perpendiculaire à la paroi intérieure et/ou perpendiculaire à la paroi extérieure.

6. Procédé selon l'une des revendications précédentes,
**caractérisé en ce qu'**au moins un élément interne est un élément de séparation (30) qui divise la cavité (24) en deux cavités partielles (34) et qui est de préférence disposé exclusivement sur la paroi extérieure.

7. Procédé selon la revendication 6,
**caractérisé en ce que** ledit au moins un élément de séparation (30) présente au moins une ouverture qui relie les deux cavités partielles (34).

8. Procédé selon la revendication 7,
**caractérisé en ce qu'**au moins un élément de soutien (26) s'étend à travers ladite au moins une ouverture dudit au moins un élément de séparation (30).

9. Procédé selon l'une des revendications 6 à 8,
**caractérisé en ce qu'**une pluralité d'éléments internes sont des éléments de séparation (30) qui s'étendent de préférence parallèlement les uns aux autres.

10. Procédé selon l'une des revendications précédentes,
**caractérisé en ce qu'**un moyen de raccordement (8) pour un composant distal de prothèse est disposé à l'extrémité distale (6) de l'emboîture de prothèse (2), et les prolongements imaginaires d'une pluralité d'éléments de soutien (26) se rejoignent dans le moyen de raccordement (8), de préférence en un point à l'intérieur du moyen de raccordement (8).

11. Procédé selon l'une des revendications précédentes,
**caractérisé en ce que** l'emboîture de prothèse (2) est une emboîture de prothèse pour une prothèse destinée à un membre inférieur, de préférence pour une prothèse de jambe.

12. Procédé selon l'une des revendications précédentes,
**caractérisé en ce que** l'emboîture de prothèse (2) comprend au moins un premier élément d'insertion (36) qui présente une surface d'appui (38) formée de manière à correspondre à la paroi intérieure de l'emboîture de prothèse.

13. Procédé selon la revendication 12,
**caractérisé en ce que** l'emboîture de prothèse (2) comprend au moins un deuxième élément d'insertion (36) qui présente une surface d'appui (38) formée de manière à correspondre à la paroi intérieure de l'emboîture de prothèse et/ou de manière à correspondre à une surface du premier élément d'insertion (36) située en face de la surface d'appui (38).
